# EUROPEAN PATENT SPECIFICATION

(11) **EP 2 092 935 B1**
(45) Date of publication and mention of the grant of the patent: **30.03.2011**
(21) Application number: 09006110.2
(22) Date of filing: 27.08.2002
(51) Int. Cl.: A61K 9/12, A61K 31/58, A61K 31/167, A61P 11/06

(54) **Pharmaceutical compositions for the treatment of asthma**
Pharmazeutische Zusammensetzungen zur Behandlung von Asthma
Compositions pharmaceutiques pour le traitement de l'asthme

(30) Priority: 28.08.2001 US 315386 P
(43) Date of publication of application: 26.08.2009
(62) Divisional of application: 02753549.1
(73) Proprietor: Schering Corporation, Kenilworth, NJ 07033-0530 (US)
(72) Inventor: Sequeira, Joel A., Ocean, NJ 07712 (US); Sharpe, Stefan A., Jersey City, NJ 07302 (US); Hart, John L., Bedminster, NJ 07921 (US)
(74) Representative: Buchan, Gavin MacNicol

(56) References cited:
- WO-A-00/44352
- WO-A-00/51591
- WO-A-00/53187
- WO-A-01/78744
- WO-A-97/47286
- WO-A-98/08519

## Description

The present invention is directed to aerosol suspension formulations which are free of chlorofluorocarbons (CFC's). More specifically, the present invention is directed to formulations that are substantially free of CFC's and formulations that have particular utility in medicinal applications, especially in metered dose pressurized inhalers (MDI's).

Metered dose inhalers have proven to be effective oral and nasal delivery systems that have been used extensively for delivering bronchodilating and steroidal compounds to asthmatics, as well as delivering other compounds such as pentamidine and non-bronchodilator anti-inflammatory drugs. The rapid onset of activity of compounds administered in this manner and the absence of any significant side effects have resulted in a large number of compounds being formulated for administration via this route. Typically, the drug is delivered to the patient by a propellant system generally comprising one or more propellants which have the appropriate vapor pressure and which are suitable for oral or nasal administration. The more preferred propellant systems typically comprise CFC propellant 11, CFC propellant 12, CFC propellant 114 or mixtures thereof. Often the vapor pressure of the propellant systems is adjusted by admixing a less volatile liquid excipient with the propellant.

However, propellants CFC 11, CFC 12 and CFC 114 belong to a class of compounds known as chlorofluorocarbons, which have been linked to the depletion of ozone in the atmosphere. It has been postulated that ozone blocks certain harmful UV rays and thus a decrease in the atmospheric ozone content will result in an increase in the incidence of skin cancer. In the 1970's certain steps were taken to reduce the CFC emissions from aerosols. Other propellants, such as hydrocarbons, were used, or the product was delivered in a different manner. Because CFC usage in medicinal applications is relatively low, i.e. less than 1% of total CFC emissions, and because of the health benefits associated with metered dose inhalers, steps were not taken at that time to restrict the use of CFC propellants in metered dose inhalers.

However, continuing and more sophisticated ozone measurements have indicated that the earlier restrictions in CFC usage were insufficient and that adddional, significant steps should be taken to drastically reduce CFC emissions. Recommendations have been made that CFC production be virtually discontinued. As a result, it may not be possible to continue to use CFC propellants in the intermediate and long term. While some efforts have been made to use non-pressurized metered dose inhalers, many of these devices have not been completely successful. Some of the performance issues related to these are: delivery of uniform doses, mechanical complexity, provision of the required doses per unit of an aerosol container, compliance with stringent regulatory standards, and difficulty for individuals to utilize because they are bulky and/or cumbersome for patient use, particularly when patient has an acute need for the medication.

As a result, there is a need for CFC-free pressurized aerosol formulations, such as metered dose inhalers, which are substantially free of CFC's, Non-CFC propellant systems must meet several criteria for pressurized metered dose inhalers. They must be non-toxic, stable and non-reactive with the medicament and the other major components in the valve/actuator. One propellant which has been found to be suitable is CF₃ CHFAF₃, also known as HFA 227, HFC 227 or 1,1,1,2,3,3,3 heptafluoropropane. From hereon forward, this propellant will be referred to as HFA 227. However, certain physical properties, i.e., polarity and solubility of HFA 227 differ from those of commonly used CFC propellants. Commonly used surfactants may be insoluble in HFA 227. Moreover, where the medicament is to be delivered as a solution, the medicament may not be readily soluble in this propellant. The polarity difference between HFA 227 and the previously used CFC propellants may result in a different delivery of the medicament when HFA 227 replaces a CFC propellant. The medicament may cream, settle or agglomerate in the non-CFC propellant even though this did not occur in the CFC propellant. Another such non-chlorofluorocarbon propellant is Hydrofluorocarbon 134a, also known as 1,1,9,2-tetrafluoroethane or HFA 134a. From hereon forward, this propellant will be referred to as HFA 134a

Prior art formulations containing mometasone in combination with HFA 227 in a metered dose inhaler utilize ethanol to suspend the mometasone in a crystalline state in combination with the propellant. These formulations have improved stability over time.

The specific combinations noted above may not provide the desired solubility, stability, low toxicity, exact dosage, correct particle size (if suspension) and/or compatibility with commonly used valve assemblies of metered dose inhalers. Accordingly, there exists a need for CFC free formulations for the treatment of asthma, and processes for producing the same, that do not suffer from the aforementioned infirmities.

### SUMMARY OF THE INVENTION

Accordingly, the present invention is directed to a process for producing non-toxic formulations that are substantially free of CFC's that have improved stability and compatibility with the medicament and which are relatively easily manufactured.

The present invention is also directed to a metered dose inhaler containing an aerosol suspension formulation for inhalation, said aerosol suspension formulation for inhalation comprising: an effective amount of mometasone furoate; an effective amount of formoterol fumarate; and 1,1,1,2,3,3,3, heptafluoropropane; wherein the ratio of mometasone furoate to formoterol fumarate is about 400 µg of mometasone furoate to about 12 µg of formoterol fumarate to about 50 µg of mometasone furoate to about 6 µg of formoterol fumarate, wherein the formoterol fumarate flocculates with the mometasone furoate, and wherein the formulation is substantially free of a carrier.

The present invention is also directed to a metered dose inhaler containing an aerosol suspension formulation for inhalation, said aerosol suspension formulation for inhalation comprising: an effective amount of mometasone furoate; an effective amount of formoterol fumarate; about 0.05% to about 0.3% by weight of a bulking agent; and 1,1,1,2-tetrafluoroethane; wherein the ratio of mometasone furoate to furmoterol fumarate is about 400 µg of mometasone furoate to about 12 µg of formoterol fumarate to about 50 µg of mometasone furoate to about 6 µg of formoterol fumarate, and wherein the formoterol fumarate flocculates with the mometasone furoate.

The present invention is also directed to a process for producing an serosol suspension formulation for inhalation, said aerosol suspension formulation for inhalation comprising: an effective amount of mometasone furoate; an effective amount of formoterol fumarate: and 1,1,1,2,3,3, heptafluoropropane; wherein the ratio of mometasone furoate to formoterol fumarate is about 400 µg of mometasone furoate to about 12 µg of formoterol fumarate to about 50 µg of mometasone furoate to about 6 µg of formoterol fumarate, and wherein the formoterol fumarate is flocculated with the mometasone furoate in said aerosol suspension formulation, and wherein the formulation is free of a bulking agent, comprising the steps of: mixing a dry powder blend of micronized mometasone furoate and formoterol fumarate with a dry powder surfactant to form a uniform mixture; filling said mixture into a metered dose inhaler canister; crimping said canister with a metering valve; and filling said canister with a nonchlorofluorocarton propellant. The present invention is also directed to the products produced by the foregoing process.

The present invention is also directed to a metered dose inhaler containing an aerosol suspension formulation for inhalation, said aerosol suspension formulation for inhalation comprising: an effective amount of mometasone furoate; an effective amount of formoterol fumarate; a dry powder surfactant; and 1,1,1,2,3,3,3, heptafluoropropane; wherein the ratio of mometasone furoate to formoterol fumarate is about 400 µg of mometasone furoate to about 12 µg of formoterol fumarate to about 50 µg of mometasone furoate to about 6 µg of formoterol fumarate, wherein the formoterol fumarate flocculates with the mometasone furoate, wherein the formulation is free of additional excipients, and wherein the metered dose inhaler emits a dose having uniform drug content upon actuation of the metered dose inhaler.

The present invention is also directed to a metered dose inhaler containing an aerosol suspension formulation for inhalation, said aerosol suspension formulation for inhalation comprising: an effective amount of mometasone furoate; an effective amount of formoterol furnarate; and 1,1,1,2,3,3,3, heptafluoropropane; wherein the ratio of mometasone furoate to formoterol fumarate is about 400 µg of mometasone furoate to about 12 µg of formoterol furnarate to about 50 µg of mometasone furoate to about 6 µg of formoterol fumarate, wherein the formoterol fumarate flocculates with the mometasone furoate, and wherein the formulation contains less than 0.1% of an epoxide degradation product of mometasone furoate.

The present invention is also directed to a metered dose inhaler containing an aerosol suspension formulation for inhalation, said aerosol suspension formulation for inhalation comprising: an effective amount of mometasone furoate; an effective amount of formoterol fumarate; and 1,1,1,2,3,3,3, heptafluoropropane; wherein the ratio of mometasone furoate to formoterol fumarate is about 400 µg of mometasone furoate to about 12 µg of formoterol fumarate to about 50 µg of mometasone furoate to about 6 µg of formoterol fumarate, wherein the formoterol fumarate flocculates with the mometasone furoate, wherein the percent of the fine particles dispensed upon actuation of the metered dose inhaler is about 55% to about 85% and wherein said fine particles have a particle size of less than about 4.7µm.

### BRIEF DESCRIPTION OF THE DRAWINGS

Figure 1 is the mometasone furoate Andersen Cascade impactor profile of the mean of three units of the formoterol fumarate/mometasone furoate 6/50 µg actuation combination inhaler with HFA 227 and no bulking agent.
Figure 2 is the formoterol fumarate Andersen Cascade Impactor profile of the mean of three units of the formoterol fumarate/mometasone furoate 6150 µg actuation combination inhaler with HFA 227 and no bulking agent.
Figure 3 is the formoterol fumarate Andersen Cascade impactor profile of the mean of three units of the formoterol fumarate/mometasone furoate 6/50 µg actuation combination inhaler and HFA 134a with low bulking agent.
Figure 4 is the mometasone furoate Andersen Cascade impactor profile of the mean of three units of the formoterol fumarate/mometasone furoate 6/50 µg actuation combination inhaler and HFA 134a with low bulking agent.

### DETAILED DESCRIPTION OF THE INVENTION

Mometasone furoate, the active component of ELOCON® lotion, cream, and ointment, is an anti-inflammatory corticosteroid having the chemical name, 9,21-Dichtoro-11 (beta), 17-dihydroxy-16(alpha)-methylpregna-1,4-diene-3,20-dione 17-(2 furoate). It is practically insoluble in water; slightly soluble in methanol, ethanol, and isopropanol; soluble in acetone and chloroform: and freely soluble in tetrahydrofuran. Its partition coefficient between octanol and water is greater than 5000. Mometasone can exist in various hydrated, crystalline and enantiomeric forms, e.g., as a monohydrate. This product is available from Schering-Plough Corporation, Kenilworth, New Jersey.

Formoterol fumarate is a selective beta 2-adrenergic bronchodilator. Its chemical name is (±)-2-hydroxy-5-[(1RS)-1-hydroxy-2-[[(1RS)-2-(4-methoxyphenyl)-1-methylethyl]- amino]ethyl]formanilide fumarate dihydrate. Formoterol fumarate is a white to yellowish crystalline powder, which is reportedly freely soluble in glacial acetic acid, soluble in methanol, sparingly soluble in ethanol and isopropanol, slightly soluble in water, and practically insoluble in acetone, ethyl acetate, and diethyl ether. Formoterol fumarate can exist in various hydrated, crystalline, and enantiomeric forms, e.g., as a monohydrate. This product is available commercially from Novartis Corporation, East Hanover, New Jersey.

The invention is of particular utility where the medicament is formoterol fumarate and mometasone furoate, or end salts, enantiomers and clathrates thereof.

The mometasone furoate and formoterol fumarate can be in a weight ratio of about 1 to 1 mometasone furoate to formoterol fumarate, or about 50 to 1 mometasone furoate to formoterol fumarate, or about 20 to 1 mometasone furoate to formoterol fumarate, or about 12 to 1 mometasone furoate to formoterol fumarate, or about 18 to 1 mometasone furoate to formoterol fumarate, or about 10 to 1 mometasone furoate to formoterol fumarate, or about 8 to 1 mometasone furoate to formoterol fumarate.

These ratios roughly equate to a dose range of 6 µg of formoterol fumarate to 50 µg of mometasone furoate per dose, or about 6 µg to 100 µg of formoterol fumarate to mometasone furoate per dose, or about 8 µg to 100 µg of formoterol fumarate to mometasone furoate per dose, or about 6 µg to 200 µg of formoterol fumarate to mometasone furoate per dose, or about 8 µg to 200 µg of formoterol fumarate to mometasone furoate per dose, or about 12 µg to 200 µg of formoterol fumarate to mometasone furoate per dose, or about, 12 µg to 400 µg of formoteral fumarale to mometasone furoate per dose.

Propedant-based pharmaceutical aerosol formulations in the art typically use a mixture of liquid chlorofluorocarbons as the propellant, although many others use a single propellant. As is known in the art, the propellant serves as a vehicle for both the active ingredients and excipients. Fluorotrichloromethane, dichlorodifluoromethane and dichlorotetrafluoroethane are the most commonly used propellants in aerosol formulations for administration by inhalation. Such chlorofluorocarbons (CFC's), however, have been implicated in the destruction of the zone layer and their production is being phased cut HFA 134a and HFA 227 are said to be less harmful to the ozone than many chlorofluorocarbon propellants, and both either individually or in combination are considered to be used within fine scope of the present invention. However, conventional chlorofluorocarbons, or mixtures thereof, may also be used as propellants for the formulations of the present invention.

As is known to one of skill in the art a carrier and/or bulking agent is an inert substance in which or on to which the active drug ingredient(s) and excipient(s) if present are dispersed. When the formulations of the present invenion utilize HFA 227 as the propellant it has been surprisingly found that a carrier is not necessary. Accordingly there is disclosed a metered dose inhaler containing an aerosol suspension formulation for inhalation, said aerosol suspension formulation for inhalation comprising: as effective amount of mometasone furoate; an effective amount of formoterol fumarate; and 1,1,1,2,3,3,3, heptafluoropropane; wherein the ratio of mometasone furoate to formoterol fumarate is about 400 µg of mometasone furoate to about 12 µg of formoterol fumarate to about 50 µg of mometasone furoate to about 6 µg of formoterol fumarate, wherein the formoterol fumarate flocculates with the mometasone furoate, and wherein the formulation is substantially free of a carrier.

The processes for producing the formulations of the present invention preferably utilize HFA 227 or HFA 134a, or a combination thereof, in combination with mometasone furoate and formoterol fumarate, optionally, a liquid excipient, and opptionally a surfactant. The excipient facilitates the compatibility of the medicament with the propellant and also lowers the discharge pressure to an acceptable range, i.e., about 2.76-5.52 X 10⁵ newton/meter² absolute (40 to 80 psi), preferably 3.45-4.83 X 10⁵ newton/meter² absolute (50 to 70 psi). The excipient chosen must be non-reactive with the medicaments, relatively non-toxic, and should have a vapor pressure below about 3.45 X 10⁵ newton/meter² absolute (50 psi).

As used hereinafter the term "medium chain fatty acids" refers to chains of alkyl groups terminating in a -COOH group and having 6-12 carbon atoms, preferably 8-10 carbon atoms. The term "short chain fatty acids" refers to chains of alkyl groups terminating in a -COOH group and having 4-8 carbon atoms. The term "alcohol" Includes C₁-C₃ alcohols, such as methanol, ethanol and isopropanol.

Among the preferred excipients are: propylene glycol diesters of medium chain fatty acids available under the tradename Miglyol 840 (from Huls America, Inc. Piscataway, N.J.); triglyceride esters of medium chain fatty adds available under the tradename Miglyol 812 (from Huts): perfluorodimethylcyclobutane available under the tradename Vertrel 245 (from E. I. DuPont de Nemours and Co. Inc. Wilmington, Del.); perfluorocyclobutane available under the tradename octafluorocyclobutane (from PCR Gainsville, Fla.); polyethylene glycol available under the tradename EG 400 (from BASF Parsippany, N.J.); menthol (from Pluess-Stauffer International Stanford, Conn.); propylene glycol monolaurate available under the tradename laurogtycol (from Gattefosse Elmsford, N.Y.); diethylene glycol monoethylether available under the tradename Transcutol (from Gattefosse); polyglycolized glyceride of medium chain fatty adds available under the tradename Labrafac Hydro WL 12t9 (from Gattefosse); alcohols, such as ethanol, methanol and isopropanol; eucalyptus oil available (from Pluses-Stauffer International); and mixtures thereof.

A surfactant is frequently included in aerosol formulations, for purposes such as assisting with maintaining a stable suspension of the drug and lubricating the metering valve. The formulation of the present invention does not required a surfactant for maintenance of ready dispersability (such as by moderate agitation immediately prior to use), as the drugs form loose floccules in the propellant and does not exhibit a tendency to settle or compact. In the case of HFA 227 upon undisturbed storage, the drug particles remain suspended in their flocculated state. Thus, a surfactant optionally may be added to lower the surface and interfacial tension between the medicaments and the propellant. Where the medicaments, propellant and excipient are to form a suspension, a surfactant may or may not be required. Where the medicament, propellant and excipient are to form a solution, a surfactant may or may not be necessary, depending in part, on the solubility of the particular medicament and excipient The surfactant may be any suitable, non-toxic compound which is non-reactive with the medicament and which substantially reduces the surface tension between the medicament, the excipient and the propellant and/or acts as a valve lubricant. Among the preferred surfactants are: oleic acid available under the tradename oleic acid NF6321 (from Henkel Corp. Emery Group, Cincinnati, Ohio); cetylpyridinium chloride (from Arrow Chemical, Inc. Westwood, N.J.); soya lecithin available under the tradename Epikuron 200 (from Lucas Meyer Decatur, III.); polyoxyethylene(20) sorbitan monolaurate available under the tradename Tween 20 (from ICI Specialty Chemicals, Wilmington. Del.); polyoxyethylene(20) sorbitan monostearate available under the tradename Tween 60 (from ICI); polyoxyethylene(20) sorbitan monooleate available under the tradename Tween 80 (from ICI); polyoxyethylene (10) stearyl ether available under the tradename Brij 76 (from ICI); polyoxyethylene (2) oleyl ether available under the tradename Brij 92 (frown ICI): Polyoxyethylene-polyoxypropylen-ethylenediamine block copolymer available under the tradename Tetronic 150 R1 (from BASF): polyoxypropylene-polyoxyethylene block copolymers available under the tradenames Pluronic L-92. Pluronic L-121 end Pluronic F 68 (from BASF); castor oil ethoxylate available under the tradename Alkasurf CO-40 (from Rhone-Poulenc Mississauga Ontario, Canada); and mixtures thereof.

As with other drugs which have slight solubility in ethanol, there is a tendency for mometasone furoate to exhibit crystal growth in ethanol-containing formulations. Formulation parameters which do not promote drug particle size growth are known. These parameters provide the advantage of minimizing the required ethanol concentrations, to reduce the potential for unpleasant taste sensations and render the compositions more suitable for use by children and others with low alcohol tolerance.

A certain minimum level of ethanol is preferred to provide consistent and predictable delivery of the drug from a metered dose dispenser. This minimum level is about 1 weight percent of the total formulation, which results in a marginally acceptable drug delivery. Increased amounts of ethanol generally improve drug delivery characteristics. However, to prevent drug crystal growth in the formulation, it is preferred to limit the concentration of ethanol. Experimental data indicate that the ratio of the weight of mometasone furoate to the weight of ethanol is important in preventing particle size increases.

The active ingredients may be put into the containers housing the formulation as follows: the container that houses the medication can be filled with medicine, ethanol and a surfactant in single or multiple steps, preferably in a single step. Similarly, the propellant or mixture of propellants may be added to the container in the same or in multiple steps. The suspensions of the formulations of the present invention contain floccules of the ingredients. A floccule is an aggregation of particles that form a lattice type of structure that resists complete settling. The loose structure of the lattice permits the aggregates to break up easily and distribute readily with a small amount of agitation. More specifically, when mometasone is suspended in a propellant, over time the particles of mometasone will tend to flocculate in the center of the suspension. These particles readily disperse upon agitation or shaking of the metered dose inhaler canister. Surprisingly, the addition of formoterol to the suspension did not after this phenomena. When the propellant is HFA 227, the formoterol fumarate and mometasone furoate form floccules in suspension such that the mometasone and formoterol are aggregated with each other. When the propellant is HFA 134a, the presence of a bulking agent or carrier such as lactose in an amount of about 0.05% to about 0.3% by weightis preferred to enhance drug delivery upon actuation of the inhaler. With 134a based formulations, the formoterol, mometasone and lactose have a tendency to sediment to the bottom of the canister because HFA 134a is less dense than HFA 227; thus, shaking of the canister to re-form the suspension prior to actuation of the meter is preferred for uniform drug delivery. Other bulking agents that may be used in HFA 134a suspensions include, for example, mannitol, glucose, sucrose and trehalose.

Formulations of the invention are made according to procedures customary in the art for other aerosol compositions. Typically, all components except the propellant are mixed and introduced into aerosol containers. The containers can then be chilled to temperatures below the boding point of the propellant, and the required amount of the chilled propellant added before the metering valve is crimped on to the container. Alternatively, the containers can be fitted with a metering valve before being filled with propellent, and the required quantity of propellant will be introduced through the valve.

The formulations of the present invention may be filled into the aerosol containers using conventional filling equipment. Since HFA 227 and HFA 134a may not be compatible with all elastomeric compounds currently utilized in present aerosol valve assemblies, it may be necessary to substitute other materials, such as white buna rubber, or to utilize excipients and optionally surfactants which mitigate the adverse effects of HFA 227 or 134a on the valve components. Suspensions of the present invention preferably may be prepared by either the pressure filling or cold filling procedures known in the art

Depending on the particular application, the container may be charged with a predetermined quantity of formulation for single or multiple dosing. Typically, the container is sized for multiple-dosing, and, therefore it is very important that the formulation delivered is substantially uniform for each dosing. For example, where the formulations is for bronchodilation, the container typically is charged with a sufficient quantity of the formulation for 200 actuations.

Suitable suspensions may be screened In part by observing several physical properties of the formulation, i.e. the rate of particle agglomeration, the size of the agglomerates and the rate of particulate creaming/setting and comparing these to an acceptable standard. Such, suitable solutions may be screened/evaluated by measuring the solubility of the medicament over the entire recommended storage temperature range.

For metered dose inhalers, suspensions may be particularly preferred for efficacy and stability considerations. Those skilled in the art may choose to add one or more preservative, buffer, antioxidant, sweetener and/or flavors or other taste masking agents depending upon the characteristics of the formulation.

The available metering valve delivery volumes range from about 25 to about 100 microliters per actuation, while the amounts of drug substance required in a dose for treating a particular condition is generally about 10 to about 500 micrograms per valve actuation. These two factors combined pose limitations that dictate the points within the foregoing ethanol parameters for a given formulation. The determination of such amounts is within the skill of workers in this art.

In formulations of the present invention which are suitable for treating lower respiratory system disorders such as asthma, at least a substantial portion of the drug is present as suspended particles having respirable sizes, e.g., about 0.5 to about 10 micrometers in their largest dimension. In formulations which are suitable for treating upper respiratory system disorders such as rhinitis, somewhat larger drug particles may be permissible, but the foregoing size range remains preferred. Where the active compound forms a suspension, the particle size should be relatively uniform, with substantially all the particles preferably ranging between about 0.1-25 microns, preferably 0.5-10 microns, more preferably 1-5 microns. Particles larger than 25 microns may be held up in the oropharyngeal cavity, while particles smaller than about 0.5 micron preferably are not utilized, since they would be more likely to be exhaled and, therefore, not reach the lungs of the patient.

Also within the scope of the present invention is methods of treating diseases of the airways susceptible to treatment with mometasone furoate and formoterol fumarate in effective amounts as set forth in examples 3, 4 and 5 below. The medicaments may be administered once or twice a day in accordance with the doses set forth in examples 3, 4 and 5.

Another aspect of the present invention comprises novel formulations comprising a dispersion system of a well mixed ternary blend of the two drug substance powders mometasone furoate and formoterol fumarate disperse with a third powder-surfactant, such as, for example lecithin, stearic acid, palmitic acid, magnesium stearate, magnesium palmitate, magnesium laureate and other suitable dry powder blend surfactants as are known to one of skill in the art.

The dry blend may be mixed for example in a Turbula Mixer T2C for about 5 minutes, or for such amount of time is known to one of skill in the art to achieve a uniform blend of the powders. This dispersion system is metered individual into each inhaler can with a powder filling instrument, such as for example by an Autodose Powdemium - One Too Many System, into 15 mL aluminum teflon coated (FPT - fluorinated ethylene copolymer) or other polymer coated, cans. The cans can then be crimped with 63 microliter valves or the like and filled with HFA-227 or HFA-134a propellant using propellant filling equipment, such as, for example, a Pamasol Model P2008/012. The cans filled with the suspension product are thereafter sonicated by a sonicator, such as, for example, a Branson 5210 sonicator for about 5 minutes as is known to one in the art.

These particular formulations allow for the manufacture of a two drug substance combination MDI that exhibits a consistent Drug Content Uniformity (DCU) without the use of additional excipients and/or additives. The use of this type of dry 2-step filling procedure precludes the possibility of crystal growth of the active ingredients during the filling process and assures a consistent particle size distribution in the product filled during the beginning, middle and end of the filling process. This formulation and filling process assure adequate dispersion of the particles in the suspending medium HFA-227, absence of crystal growth, absence of caking and adequate drug content uniformity upon delivery of the dose.

Certain aspects of the invention are further described in the following examples. In the examples, "percent" indicates weight percentage unless the context dearly indicates otherwise. The examples below further describe the present invention.

The following dry powder blend samples were prepared.

### Example 1

| **Table 1. Dry Powder Blends of Mometasone Furoate (91%), Formoterol Fumarate (9%) & Lecithin (0.1%, 0.01% and 0.02%)*** | | | | |
|---|---|---|---|---|
| **Mometasone Furoate (mg)** | **Formoterol Fumarate (mg)** | **Lecithin (mg)** | **Total Weight of Blend (mg)** | **Weight Per Can (mg)** |
| 616.0 | 61.70 | 0.686 | 678.4 | 13.57 |
| 621.0 | 62.00 | 0.070 | 683.1 | 13.66 |
| 621.0 | 61.80 | 0.144 | 682.9 | 13.66 |
| *: All weights presented on the w/w basis in the ternary blend. | | | | |

As is apparent, the weigh ratio of mometasone furoate to formoterol fumarate is roughly about 10 to 1. To prepare, directly mix a dry powder blend of the mometasone furoate, formoterol fumarate and lecithin in a Turbula mixer for about 5 minutes in the above identified amounts. Thereafter, meter the mixtures into the 15 mL canister using an Autodose Powdemium powder filling instrument or the like. Thereafter, crimp with a 63 microliter valve and add the propellant up to about 10 g/can. Then, sonicate for 5 minutes.

### Example 2

| **Table 2. MDI Formulation Blends of Mometasone Furoate, Formoterol Fumarate, Lecithin and HFA-227°** | | | |
|---|---|---|---|
| **Mometasone Furoate (%)** | **Formoterol Fumarate (%)** | **Lecithin (%)** | **HFA-227 (%)** |
| 0.1 | 0.01 | 0.01 | 99.88 |
| 0.1 | 0.01 | 0.001 | 99.89 |
| 0.1 | 0.01 | 0.002 | 99.89 |
| *: All weights presented on the w/w basis in the finished product. | | | |

Table 2 describes the various amounts of the active ingredients and surfactant when combined with HFA-227 in the finished metered dose inhaler canister.

Certain other aspects of the invention are further described in the following examples. Again, in the examples, "percent" indicates weight percentage unless the context dearly indicates otherwise. The examples below further describe the present invention.

Examples 3. 4 and 5 provide examples of the varying amounts of various ingredients of the formulations of the present invention:

### Example 3

| **Formulation Prototype (Drug:Drug) Ratio** | **Mometasone Furoate (%)** | **Formoterol Fumarate (%)** | **Oleic Acid (%)** | **Ethanol (%)** | **HFA-227 (%)** |
|---|---|---|---|---|---|
| A (100 µg:6 µg) | 0.112 | 0.009 | 0.001 | 2.378 | 97.5 |
| B (50 µg:6 µg) | 0.056 | 0.007 | 0 | 2.437 | 97.5 |
| C (100 µg:8 µg) | 0.112 | 0.009 | 0.011 | 2.368 | 97.5 |
| D (200 µg:12 µg) | 0.224 | 0.014 | 0.011 | 2.251 | 97.5 |
| E (100 µg:8 µg) | 0.112 | 0.009 | 0 | 2.379 | 97.5 |
| F (50 µ:6 µ) | 0.056 | 0.007 | 0.001 | 2.436 | 97.5 |
| G (50 µg:8 µg) | 0.056 | 0.007 | 0.011 | 2.426 | 97.5 |
| H (50 µg:6 µg) | 0.056 | 0.007 | 0.011 | 1.5 | 98.426 |
| I (50 µg:6 µg) | 0.056 | 0.007 | 0.011 | 1.75 | 98.176 |
| J (50 µg:6 µg) | 0.056 | 0.007 | 0.0 | 1.5 | 98.437 |
| K (50 µg:6 µg) | 0.056 | 0.007 | 0.0 | 1.75 | 98.187 |
| L (200 µg:12 µg) | 0.224 | 0.014 | 0.011 | 1.5 | 98.251 |
| M (200 µg:12 µg) | 0.224 | 0.014 | 0.011 | 1.75 | 98.001 |
| N (200 µg:12 µg) | 0.224 | 0.014 | 0.0 | 1.5 | 98.282 |
| O (200 µg:12 µg) | 0.224 | 0.014 | 0.0 | 1.75 | 98.012 |

### Example 4

| **Formulation Prototype (Drug:Drug)** **Ratio.** | **Mometasone Furoate (%)** | **Formtoterol Fumarate (%)** | **Oleic Acid (%)** | **Ethanol (%)** | **HFA-134a (%)** |
|---|---|---|---|---|---|
| A (100 µg:8 µg) | 0.112 | 0.009 | 0.001 | 2.378 | 97.5 |
| B(50 µg:6 µg) | 0.056 | 0.007 | 0 | 2.437 | 97.5 |
| C (100 µg:8 µg) | 0.112 | 0.009 | 0.011 | 2.388 | 97.5 |
| D (200 µg:12 µg) | 0.224 | 0.014 | 0.011 | 2.251 | 97.5 |
| E (100 µg:8 µg) | 0.112 | 0.009 | 0 | 2.379 | 97.5 |
| F (50 µg:6 µ) | 0.056 | 0.007 | 0.001 | 2.436 | 97.5 |
| G (50µg:6 µg) | 0.058 | 0.007 | 0.011 | 2.426 | 97.5 |
| H (50 µg:6 µg) | 0.058 | 0.007 | 0.011 | 1.5 | 98.426 |
| I (50 µg:6 µg) | 0.056 | 0.007 | 0.011 | 1.75 | 98.176 |
| J (50 µg:6 µg) | 0.056 | 0.007 | 0.0 | 1.5 | 98.437 |
| K (50 µg:8 µg) | 0.056 | 0.007 | 0.0 | 1.75 | 98.187 |
| L (200 µg:12 µg) | 0.224 | 0.014 | 0.011 | 1.5 | 98251 |
| MP (200 µg:12 µg) | 0.224 | 0.014 | 0.011 | 1.75 | 98.001 |
| N (200 µg:12 µg | 0.224 | 0.014 | 0.0 | 1.5 | 98.262 |
| O (200 µg: 12 µg) | 0.224 | 0.014 | 0.0 | 1.75 | 98.012 |

### Example 5

| **Formulation Prototype (Drug:Drug) Ratio** | **Mometasone Furoate (%)** | **Formoterol Fumarate (%)** | **Oleic Acid (%)** | **Ethanol (%)** | **HFA-227/HFA-134a (50:50) (%)** |
|---|---|---|---|---|---|
| A (100 µg:8 µg) | 0.112 | 0.009 | 0.001 | 2.378 | 97.5 |
| B (50 µg:6 µg) | 0.068 | 0.007 | 0 | 2.437 | 97.5 |
| C (100 µg:8 µg) | 0.112 | 0.009 | 0.011 | 2.368 | 97.5 |
| D (200 µg:12 µg) | 0.224 | 0.014 | 0.011 | 2.251 | 97.5 |
| E (100 µg:8 µg) | 0.112 | 0.009 | 0 | 2.379 | 97.5 |
| F (50 µg:6 µ) | 0.058 | 0.007 | 0.001 | 2.438 | 97.5 |
| G (50 µg:6 µg) | 0.058 | 0.007 | 0.011 | 2.426 | 97.5 |
| H (50 µg:6 µg) | 0.056 | 0.007 | 0.011 | 1.5 | 98.425 |
| I (50 µg:6 µg) | 0.056 | 0.007 | 0.011 | 1.75 | 9A.176 |
| J (50 µg:6 µg) | 0.056 | 0.007 | 0.0 | 1.5 | 98.437 |
| K (50 µg µg) | 0.056 | 0.007 | 0.0 | 1.75 | 98.187 |
| L (200 µg:12 µg) | 0.224 | 0.014 | 0.011 | 1.5 | 98.251 |
| M (200 µg:12 µg) | 0.214 | 0.014 | 0.011 | 1.75 | 98.001 |
| N (200 µg:12 µg) | 0.224 | 0.014 | 0.0 | 1.5 | 98.262 |
| O (200 µg:12 µg) | 0.224 | 0.014 | 0.0 | 1.75 | 98.012 |

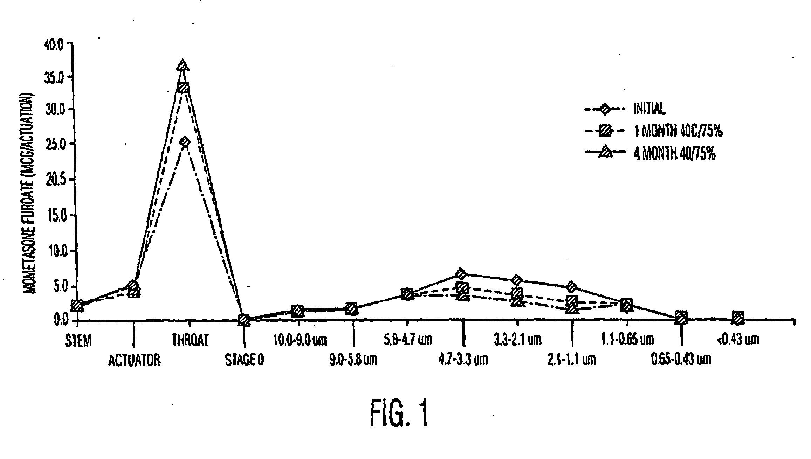

The formulations of the present invention were further analyzed using an Andersen Cascade Impactor as described In Examples 6 and 7. The Andersen Cascade impactor is widely used for measuring the particle size distribution of airborne particles and more specifically pharmaceutical aerosols. The eight stage Andersen impactor separates the sample into nine size intervals when used with a backup filter after the last Impaction stage. In Figure 1 to 4, this corresponds Stage 0 of particles having a particle size less than 10 µm to a particle size of 0.65-0.43 µm in stage 8, with the final stage corresponding to the filter for particles less than 0.43 µm. The measurement of the particle size at the throat" in the figures corresponds to the entry port of the Impactor. The fine particle fraction is defined as the percentage of particles having a particle size of less than 4.7 µm. The fine particle dose is defined as the amount in µg per dose that is less than 4.7 µm in size in each actuation. The µg/shot is the total amount of emitted drug product that exits the metered dose inhaler upon actuation. The determination of the particle size distribution of the emitted dose of the formulation using an Andersen Cascade Impactor is known to one of skill in the art.

### Example 6

### Andersen Cascade Impactor data on Formoterol/Mometasone 6/50 µg/actuation combination inhaler- HFA 227, without bulking agent system

| | **Mometasone Furoate** | | | | | |
|---|---|---|---|---|---|---|
| | **initial** | **1 Month** | | **3 Month** | | **4 Month** |
| | - | 4C/40C | 40C/75% | 4C/40C | 25C/60% | 40C/75% |
| Fine Portion Fraction % | 36.5 | 16.4 | 26.3 | 9.0 | 30.4 | 18.6 |
| Fine Particle Dose (µg/shot) | 19.7 | 9.2 | 13.9 | 5.3 | 16.1 | 10.4 |
| MMAD (microns) | 3.7 | 5.0 | 4.1 | 8.3 | 4.2 | 4.6 |
| µg/shot (metered dose) | 82.9 | 62.6 | 62.4 | 60.5 | 61.5 | 63.3 |

| | **Formoterol Fumarate** | | | | | |
|---|---|---|---|---|---|---|
| | **Initial** | **1 Month** | | **3 Month** | | **4 Month** |
| | - | 4C/40C | 40C/75% | 4C/40C | 25C/60% | 40C/75% |
| Fine Particle Fraction % | 48.3 | 39.0 | 41.1 | 35.2 | 4.3 | 40.1 |
| Fine Particle Dose (µg/shot) | 2.6 | 2.2 | 2.4 | 1.9 | 2.6 | 2.2 |
| MMAD (microns) | 3.2 | 3.3 | 3.2 | 3.3 | 3.2 | 3.4 |
| µg /shot (metered dose) | 6.6 | 6.3 | 6.6 | 6.3 | 6.5 | 6.5 |

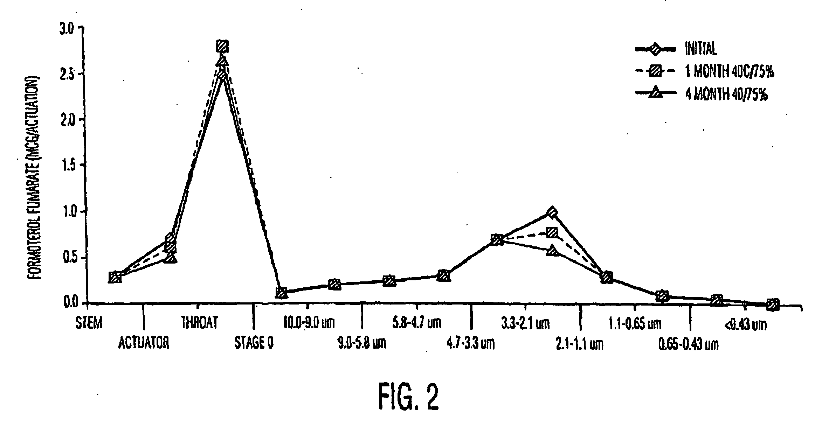

A formulation containing 6 µg of formoterol fumarate and 50 µg of mometasone furoate using HFA 227 in the absence of a bulking agent or carrier in a metered dose inhaler was analyzed by an Andersen Cascade Impactor to analyze the amount of active drug ingredients and the particle size of the actives exiting the Inhaler over time. As can be seen, the fine particle dose of the mometasone and formoterol over time and through temperature cycling under the conditions specified was maintained within acceptable limits (19.7 to 10.4 for mometasone and 2.6 to 22 for formoterol at the 4 month point). The metered dose for both actives over time was also within acceptable limits (62.9 to 63.3 for mometasone and 6.6 to 6.5 for formoterol at the 4 month point). This data corresponds to the particle size distribution for mometasone end formoterol set forth in figures 1 and 2, respectively. Thus data Indicates improved and acceptable drug delivery of the two actives.

While the fine particle fraction of mometasone furoate appeared to decrease by nearly haff, this was attributed to the coarseness of the grade of mometasone furoate used. There Is a rank order correlation of the qua6ty of the product with a decrease in the size range of the corresponding drug substance suspended in the product, It was determined that drug substance containing a high proportion of large crystals that are greater than 5 to 10 microns produces a product with an aerodynamic particles size distribution that is outside the range of a typical efficacious topical lung medication. The product containing coarser drug product also shows unacceptable particle growth with time and temperature.

A finer particle size distribution of the mometasone furoate improves the fine particle fraction of the formulation exiting the inhaler upon actuation of the metered dose inhaler. Indeed, the grade of mometasone used in the above example had a percent change in fine particle size of about 50% after two weeks of temperature cycling at -10 °C and 40 °C. However, with a similar mdi using mometasone furoate alone, but with a finer grade of mometasone furoate, it showed only about a 15% or less change in fine particle size under the same cycling conditions. This results in an increase in the fine particle fraction with regards to the mometasone, and thus improved drug delivery of the mometasone. Thus, it has been found that when a finer particle size grade of the drug substance is used, a product is produced which has suspended drug particles which do not exhibit particle growth with time and temperature. The aerodynamic particle size distribution is well within the range of a typical efficacious topical lung medication, e.g., greater than 50% of the particles are less than 4.7 microns. It also shows no significant particle growth with time and temperature.

In the case of the oral MDI containing mometasone furoate, an example of an acceptable product profile for the 100 µg /actuation strength, using an Andersen Cascade Impactor and 1-liter entry port, is given below. It should be noted that the data is based on two actuations of the metered dose inhaler.

**Table 1**

| **ANDERSEN CASCADE IMPACTOR STAGE** | **AMOUNT OF PARTICLE DEPOSITED ON PLATES** |
|---|---|
| Group 1- Entry port + Stage 0 | 8-22 µg |
| Group 2 - Stage 1 + Stage 2 | 12-21 µg |
| Group 3 - Stage 3 + Stage 4 | 122-140 µg |
| Group 4 - Stage 5-Filter | 22-41 µg |

The percentage of fine particles in group 1 ranges from about 4.9% to about 9.8%. The percentage of fine particles in group 2 ranges from about 7.3% to about 9.4%. The percentage of fine particles in stage 3 to the filter (groups 3 and4) should preferably be in a range of about 55% to about 85% where the fine particles have a particle size of less than about 4.7µm, preferably 65% to 80%, or about 80%, or about 85%, and about 81% to about 89% based upon data from above table. Finally, the percentage of fine particles in group 4 ranges from about 13.4% to about 18.3%.

The size of the suspended mometasone furoate drug contained in the drug product may be controlled in various ways. The drug substance may be more efficiently milled prior to product batch manufacture. This could include reducing the micronization feed rate, employing centrifugal classification to remove larger particles and increasing the number of cycles the material is fed into the micronizer e.g., double micronizing. Alternatively, the drug substance may be spray dried prior to product batch manufacture, for example, by super critical fluid technology, to create uniformly small drug substance particles. Further the method of manufacture can be modified, e.g., by reducing the temperature of batch manufacture, reducing the level of alcohol used to prepare the drug concentrate, or reducing the homogenization time. Finally, other processes of controlling drug substance particle size that are known in the art, e.g., using surfactants or other particle size growth retardation approaches may also be used.

### Example 7

### Andersen Cascade impactor data on Formoterol/Mometasone 6/50 µg/actuation combination Inhaler- HFA 134a, low bulking agent system

| | **Mometasone Furoate** | | | | | |
|---|---|---|---|---|---|---|
| | Initial | Month | | 3 Month | | 4 Month |
| | - | 4C/40C | 40C/75% | 4C/40C | 25C/80% | 40C/75% |
| Fine Particle Fraction % | 25.7 | 20.1 | 20.1 | 20.0 | 22.6 | 17.7 |
| Fine Particle Dose (µg/shot) | 13.6 | 10.6 | 10.1 | 10.0 | 11.4 | 8.8 |
| MMAD (microns) | 3.9 | 4.3 | 4.3 | 4.6 | 4.3 | 4.5 |
| µg /shot (metered dose) | 64.1 | 65.0 | 622 | 62.6 | 62.1 | 63.7 |

| | **Formoterol Fumarate** | | | | | |
|---|---|---|---|---|---|---|
| | Initial | 1-Month | | 3 Month | | 4 Month |
| | | 4C/40C | 40C/75% | 4C/40C | 25C/60% | 40C/75% |
| Fine Particle Fraction % | 42.5 | 38.0 | 41.3 | 40.2 | 43.6 | 40.3 |
| Fine Particle Dose (µg/shot) | 2.4 | 20 | 2.1 | 2.2 | 2.3 | 2.1 |
| MMAD (microns) | 2.9 | 3.1 | 2.9 | 32 | 3-0 | 3.1 |
| /shot (metered dose) | 6.9 | 6.5 | 6.3 | 6.8 | 6.7 | 6.6 |

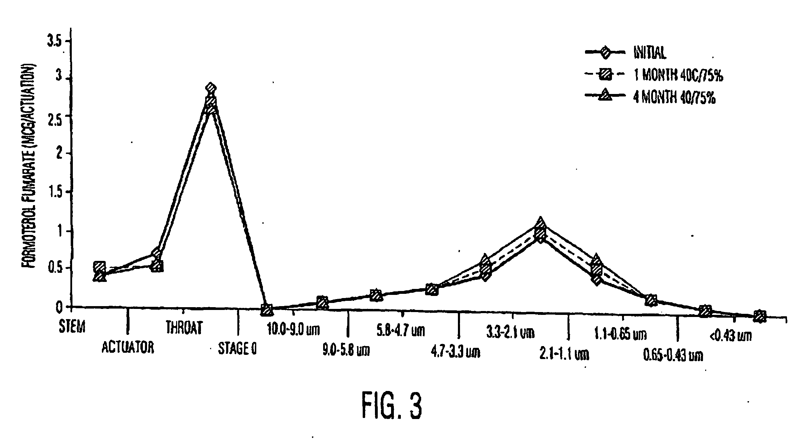

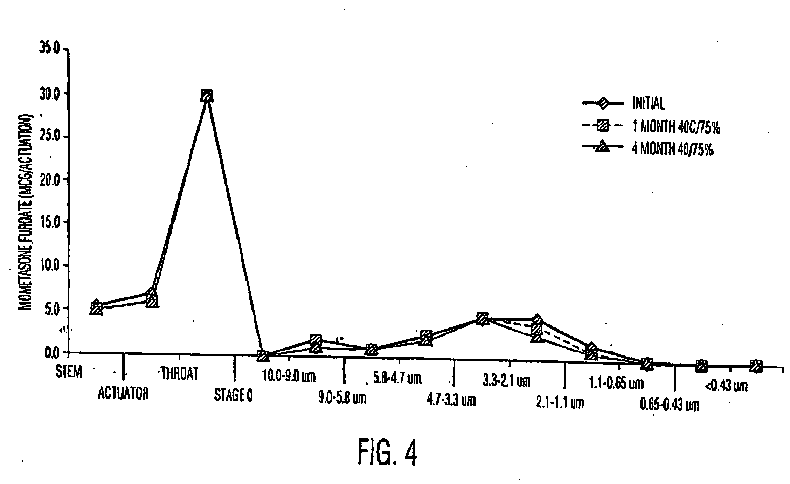

A formulation containing 6 µg of formoterol fumarate and 50 µg of mometasone furoate using HFA 134a with lactose as a bulking agent in a metered dose inhaler was analyzed by an Andersen Cascade Impactor to analyze the amount of active drug ingredients and the particle size of the actives exiting the inhaler over time. As can be seen, the fine particle dose of the mometasone and formoterol over time and through temperature cycling was maintained within acceptable limits (13.6 to 8.8 for mometasone and 2.4 to 2.1 for formoterol). Additionally, the metered dose actually delivered was also maintained over time within acceptable limits (64.1 to 63.7 for mometasone, 6.9 to 6.6 for formoterol). These data correspond to the particle size distribution for formoterol and mometasone set forth in figures 3 and 4, respectivety. The fine particle fraction for both mometasone and formoterol was maintained within acceptable limits. These data also indicate improved drug delivery of the two actives via mitered dose inhalation. Additionally, as set forth in example 6, with a similar mdi using mometasone furoate alone, but with a finer grade of mometasone furoate showed only about a 15% or less change in fine particle size under the same cycling conditions. This results in an increase in the fine particle fraction with regards to the mometasone, and thus improved drug delivery of the mometasone.

### Example 8

The degradation products of the formulations of the present invention were analyzed at 4 months time at 40 °C and 75% relative humidity.

| **IMPURITY** | **HFA 277, NON-BULKED** | **HFA 134A, LOW BULKED** |
|---|---|---|
| Formoterol deg. produce-2566 | 0.10 | 0.21 |
| Total formoterol known deg. w/o SCH-2566 | 0.10 | 0.09 |
| Total formoterol unknown degradation | 0.13 | 0.38 |
| Total formoterol degradation w/o XSCH-2566 | 0.23 | 0.47 |
| Compound E | 0.06 | 0.23 |
| Total mometasone known deg. w/o Compound E | 0 | 0 |
| Total mometasone unknown degradation | 0.13 | 0.13 |
| Total mometasone degradation | 0.19 | 0.35 |
| Total related subs | 0.51 | 1.03 |

As can be seen, the non-bulked formulations containing the HFA 227 had substantially less degradation products as a whole as compared to the bulked formulations containing the HFA 134a. Specifically, the HFA 227 formulations contained less than 0.1% of a degradation product called compound E which is an epoxide type degradant associated with mometasone furoate. Formulations containing mometasone furoate containing less than 0.1% of compound E meet FDA specifications for the presence of this particular compound in inhalable formulations containing mometasone furoate.

### Example 9

### Drug Content Uniformity of Formoterol/Mometasone 6/50 µg/actuation combination inhaler- HFA 227, no bulking agent system

| | **Mometasone Furoate** | | |
|---|---|---|---|
| | initial | 1 Month | |
| | - | 4C/40C | 40C/75% |
| Overall Mean µg/shot | 54.7 | 55.6 | 54.9 |
| Relative Standard Deviation | 11.5 | 9.6 | 6.1 |

| | **Formoterol Fumarate** | | |
|---|---|---|---|
| | initial | 1 Month | |
| | - | 4C/40C | 40C/75% |
| Overall Mean µg/shot | 5.7 | 5.5 | 5.7 |
| Relative Standard Deviation | 11.6 | 10.4 | 6.8 |

### Example 10

### Drug Content Uniformity of Formoterol/Mometasone 6/50 µg/actuation combination Inhaler-HFA 134a, low bulking agent system

| | **Mometasone Furoate** | | |
|---|---|---|---|
| | Initial | 1 Month | |
| | | - 4C/40C | 40C/75% |
| Overall Mean µg/shot Relative | 59.1 | 55.7 | 57.0 |
| Standard Deviation | 17.9 | 10.9 | 19.6 |

| | **Formoterol Fumarate** | | |
|---|---|---|---|
| | Initial | 1 Month | |
| | - | 4C/40C | 40C/75% |
| Overall Mean µg/shot | 6.2 | 5.6 | 6.0 |
| Relative Standard Deviation | 17.3 | 11.2 | 26.1 |

The Drug Content Uniformity (DCU) of the inhaler was measured throughout the life of a 120 dose MDI to ascertain whether there was a consistency of dose of the active ingredients throughout the life of the product. Five canisters of each formulation (HFA 227 or HFA 134a) were analyzed and each canister delivered 120 actuations of medication and the amount of actives exiting the inhaler per actuation was measured at varying actuations, e.g., numbers 11, 12, 13, 14, (beginning) 59, 60, 61, 62 (middle) and 117, 118, 119 and 120 (end). Means were determined for the beginning, middle and end actuations, and an overall mean was determined and set forth in Examples 9 and 10 above. While the HFA 227 formulation had a lower relative standard deviation for the amount of drug emitted throughout the life of the MDI, both formulations yielded acceptable results for drug content uniformity over time.

## Claims

1. A metered dose inhaler containing an aerosol suspension formulation for inhalation, said aerosol suspension formulation for inhalation comprising: an effective amount of mometasone furoate; an effective amount of formoterol fumarate; and 1,1,1,2,3,3,3,-heptafluoropropane as the propellant; wherein the weight ratio of mometasone furoate to formoterol fumarate is from about 1 to 1 to about 50 to 1, wherein the formoterol fumarate flocculates with the mometasone furoate, and wherein the formulation is substantially free of a carrier.

2. The metered dose inhaler containing an aerosol suspension formulation for inhalation of claim 1, wherein the weight ratio of mometasone furoate to formoterol fumarate is about 10 to 1.

3. The metered dose inhaler containing an aerosol suspension formulation for inhalation of claim 1, wherein the weight ratio of mometasone furoate to formoterol fumarate is about 20 to 1.

4. The metered dose inhaler containing an aerosol suspension formulation for inhalation of claim 1, wherein the weight ratio of mometasone furoate to formoterol fumarate is about 50 to 1.

5. The metered dose inhaler containing an aerosol suspension formulation for inhalation according to any one of the preceding claims further comprising a surfactant.

6. The metered dose inhaler containing an aerosol suspension formulation for inhalation according to any one of claims 1 to 5 wherein the formulation comprises a dry powder surfactant and is free of additional excipients, and wherein the metered dose inhaler emits a dose having uniform drug content upon actuation of the metered dose inhaler.

7. The metered dose inhaler containing an aerosol suspension formulation for inhalation according to any one of claims 1 to 5, wherein the formulation contains less than 0.1 % of an epoxide degradation product of mometasone furoate.

8. The metered dose inhaler containing an aerosol suspension formulation for inhalation, according to any one of claims 1 to 5 wherein the percent of the fine particles dispensed upon actuation of the metered dose inhaler is about 55% to about 85%, and wherein said fine particles have a particle size of less than about 4.7 µm.

9. The metered dose inhaler containing an aerosol suspension formulation for inhalation according to claim 8, wherein the percent of the fine particles dispensed upon actuation of the metered dose inhaler is about 65% to about 80%, and wherein said fine particles have a particle size of less than about 4.7 µm.

10. A process for producing an aerosol suspension formulation for inhalation, said aerosol suspension formulation for inhalation comprising:
an effective amount of mometasone furoate; an effective amount of formoterol fumarate; and 1,1,1,2,3,3,3,-heptafluoropropane; wherein the weight ratio of mometasone furoate to formoterol fumarate is from about 1 to 1 to about 50 to 1, wherein the formoterol fumarate is flocculated with the mometasone furoate in said aerosol suspension formulation, and wherein the formulation is free of a bulking agent, comprising the steps of:
a) mixing a dry powder blend of micronized mometasone furoate and formoterol fumarate and, optionally, a dry powder surfactant to form a uniform mixture;
b) filling said mixture into a metered dose inhaler canister;
c) crimping said canister with a metering valve; and
d) filling said canister with 1,1,1,2,3,3,3-heptafluoropropane as the nonchlorofluorocarbon propellant.

11. The process according to claim 10, wherein the dry powder surfactant is selected from the group consisting of lecithin, stearic acid, palmitic acid, magnesium stearate, magnesium palmitate, and magnesium laureate.

12. The aerosol suspension formulation as defined in any of claims 1 to 9 or prepared as defined in any of claims 10 to 11 for treating allergic and inflammatory reactions in the respiratory tract, comprising administering by inhalation the aerosol suspension formulation.

13. The use of an aerosol suspension formulation as defined in any of claims 1 to 9 or prepared as defined in any of claims 10 to 11 for the preparation of a medicament for treating allergic and inflammatory reactions in the respiratory tract, comprising administering by inhalation the aerosol suspension formulation.

## Patentansprüche

1. Ein Dosierinhalator, der eine Aerosolsuspensionsformulierung zur Inhalation enthält, wobei die Aerosolsuspensionsformulierung zur Inhalation enthält: eine wirksame Menge an Mometasonfuroat, eine wirksame Menge an Formoterolfumarat und 1,1,1,2,3,3,3-Heptafluorpropan als Treibmittel, wobei das Gewichtsverhältnis von Mometasonfuroat zu Formoterolfumarat etwa 1 zu 1 bis etwa 50 zu 1 beträgt, wobei das Formoterolfumarat mit dem Mometasonfuroat ausflockt, und wobei die Formulierung im Wesentlichen frei von einem Träger ist.

2. Der Dosierinhalator, der eine Aerosolsuspensionsformulierung zur Inhalation enthält, nach Anspruch 1, wobei das Gewichtsverhältnis von Mometasonfuroat zu Formoterolfumarat etwa 10 zu 1 beträgt.

3. Der Dosierinhalator, der eine Aerosolsuspensionsformulierung zur Inhalation enthält, nach Anspruch 1, wobei das Gewichtsverhältnis von Mometasonfuroat zu Formoterolfumarat etwa 20 zu 1 beträgt.

4. Der Dosierinhalator, der eine Aerosolsuspensionsformulierung zur Inhalation enthält, nach Anspruch 1, wobei das Gewichtsverhältnis von Mometasonfuroat zu Formoterolfumarat etwa 50 zu 1 beträgt.

5. Der Dosierinhalator, der eine Aerosolsuspensionsformulierung zur Inhalation enthält, gemäß einem der vorhergehenden Ansprüche, ferner enthaltend ein Tensid.

6. Der Dosierinhalator, der eine Aerosolsuspensionsformulierung zur Inhalation enthält, gemäß einem der Ansprüche 1 bis 5, wobei die Formulierung ein Trockenpulvertensid enthält und frei von weiteren Hilfsstoffen ist, und wobei der Dosierinhalator bei Betätigung des Dosierinhalators eine Dosis mit einheitlichem Arzneistoffgehalt abgibt.

7. Der Dosierinhalator, der eine Aerosolsuspensionsformulierung zur Inhalation enthält, gemäß einem der Ansprüche 1 bis 5, wobei die Formulierung weniger als 0,1% eines Epoxidzersetzungsprodukts von Mometasonfuroat enthält.

8. Der Dosierinhalator, der eine Aerosolsuspensionsformulierung zur Inhalation enthält, gemäß einem der Ansprüche 1 bis 5, wobei der Prozentsatz der feinen Teilchen, die bei Betätigung des Dosierinhalators abgegeben werden, etwa 55% bis etwa 85% beträgt, und wobei die feinen Teilchen eine Teilchengröße von weniger als etwa 4,7 µm besitzen.

9. Der Dosierinhalator, der eine Aerosolsuspensionsformulierung zur Inhalation enthält, gemäß Anspruch 8, wobei der Prozentsatz der feinen Teilchen, die bei Betätigung des Dosierinhalators abgegeben werden, etwa 65% bis etwa 80% beträgt, und wobei die feinen Teilchen eine Teilchengröße von weniger als etwa 4,7 µm besitzen.

10. Ein Verfahren zur Herstellung einer Aerosolsuspensionsformulierung zur Inhalation, wobei die Aerosolsuspensionsformulierung zur Inhalation enthält:
eine wirksame Menge an Mometasonfuroat, eine wirksame Menge an Formoterolfumarat und 1,1,1,2,3,3,3-Heptafluorpropan, wobei das Gewichtsverhältnis von Mometasonfuroat zu Formoterolfumarat etwa 1 zu 1 bis etwa 50 zu 1 beträgt, wobei das Formoterolfumarat mit dem Mometasonfuroat in der Aerosolsuspensionsformulierung ausgeflockt ist, und wobei die Formulierung im Wesentlichen frei von einem Füllstoff ist, umfassend die Schritte:
a) Vermischen einer Trockenpulvermischung aus mikronisiertem Mometasonfuroat und Formoterolfumarat und gegebenenfalls einem Trockenpulvertensid, um eine einheitliche Mischung zu bilden,
b) Einfüllen der Mischung in einen Dosierinhalatorkanister,
c) Eindrücken eines Dosierventils in den Kanister und
d) Befüllen des Kanisters mit 1,1,1,2,3,3,3-Heptafluorpropan als Nicht-Fluorchlorkohlenwasserstoff-Treibmittel.

11. Das Verfahren gemäß Anspruch 10, wobei das Trockenpulvertensid ausgewählt ist aus der Gruppe, bestehend aus Lecithin, Stearinsäure, Palmitinsäure, Magnesiumstearat, Magnesiumpalmitat und Magnesiumlaureat.

12. Die Aerosolsuspensionsformulierung wie in einem der Ansprüche 1 bis 9 definiert oder hergestellt wie in einem der Ansprüche 10 bis 11 definiert zur Behandlung allergischer und entzündlicher Reaktionen im Respirationstrakt, umfassend die Verabreichung der Aerosolsuspensionsformulierung durch Inhalation.

13. Die Verwendung einer Aerosolsuspensionsformulierung wie in einem der Ansprüche 1 bis 9 definiert oder hergestellt wie in einem der Ansprüche 10 bis 11 definiert zur Herstellung eines Medikaments zur Behandlung allergischer und entzündlicher Reaktionen im Respirationstrakt, umfassend die Verabreichung der Aerosolsuspensionsformulierung durch Inhalation.

## Revendications

1. Inhalateur doseur contenant une formulation en suspension aérosol pour l'inhalation, ladite formulation en suspension aérosol pour inhalation comprenant: une quantité efficace de furoate de mométasone; une quantité efficace de fumarate de formotérol et du 1,1,1,2,3,3,3,-heptafluoropropane en tant qu'agent propulseur; dans lequel le rapport pondéral du furoate de mométasone au fumarate de formotérol est d'environ 1 à 1 à environ 50 à 1, où le fumarate de formotérol flocule avec le furoate de mométasone et où la formulation est sensiblement sans véhicule.

2. Inhalateur doseur contenant une formulation en suspension aérosol pour inhalation de la revendication 1, dans lequel le rapport pondéral du furoate de mométasone au fumarate de formotérol est d'environ 10 à 1.

3. Inhalateur doseur contenant une formulation en suspension aérosol pour inhalation de la revendication 1, dans lequel le rapport pondéral du furoate de mométasone au fumarate de formotérol est d'environ 20 à 1.

4. Inhalateur doseur contenant une formulation en suspension aérosol pour inhalation de la revendication 1, dans lequel le rapport pondéral du furoate de mométasone au fumarate de formotérol est d'environ 50 à 1.

5. Inhalateur doseur contenant une formulation en suspension aérosol pour inhalation selon l'une quelconque des revendications précédentes, comprenant en outre un tensioactif.

6. Inhalateur doseur contenant une formulation en suspension aérosol pour inhalation selon l'une quelconque des revendications 1 à 5, dans lequel la formulation comprend un tensioactif en poudre sèche et est sans excipient supplémentaire et dans lequel l'inhalateur doseur émet une dose ayant une teneur uniforme en médicament à l'actionnement de l'inhalateur doseur.

7. Inhalateur doseur contenant une formulation en suspension aérosol pour inhalation selon l'une quelconque des revendications 1 à 5, dans lequel la formulation contient moins de 0,1 % d'un produit de dégradation époxyde de furoate de mométasone.

8. Inhalateur doseur contenant une formulation en suspension aérosol pour inhalation selon l'une quelconque des revendications 1 à 5, dans lequel le pourcentage de particules fines distribuées à l'actionnement de l'inhalateur doseur est d'environ 55% à environ 85% et dans lequel lesdites particules fines ont une grosseur de particule de moins d'environ 4,7 µm.

9. Inhalateur doseur contenant une formulation en suspension aérosol pour inhalation selon la revendication 8, dans lequel le pourcentage de particules fines distribuées à l'actionnement de l'inhalateur doseur est d'environ 65% à environ 80% et dans lequel lesdites particules fines ont une grosseur de particule de moins d'environ 4,7 µm.

10. Procédé de production d'une formulation en suspension aérosol pour inhalation, ladite formulation en suspension aérosol pour inhalation comprenant:
une quantité efficace de furoate de mométasone; une quantité efficace de fumarate de formotérol et du 1,1,1,2,3,3,3,-heptafluoropropane; dans lequel le rapport pondéral du furoate de mométasone au fumarate de formotérol est d'environ 1 à 1 à environ 50 à 1, dans lequel le fumarate de formotérol flocule avec le furoate de mométasone dans ladite formulation en suspension aérosol et dans lequel la formulation est sans agent de charge, comprenant les étapes consistant à:
a) mélanger un mélange de poudre sèche de furoate de mométasone et de fumarate de formotérol micronisés et, optionnellement, un tensioactif en poudre sèche pour former un mélange uniforme;
b) remplir une boîte d'inhalateur doseur avec ledit mélange;
c) sertir ladite boîte avec une soupape de dosage et
d) remplir ladite boîte avec du 1,1,1,2,3,3,3,-heptafluoropropane en tant qu'agent propulseur non au chlorofluorocarbone.

11. Procédé selon la revendication 10, dans lequel le surfactant en poudre sèche est sélectionné parmi le groupe consistant en lécithine, acide stéarique, acide palmitique, stéarate de magnésium, palmitate de magnésium et lauréate de magnésium.

12. Formulation en suspension aérosol telle que définie dans l'une quelconque des revendications 1 à 9 ou préparée comme il est défini dans l'une quelconque des revendications 10 à 11, pour traiter des réactions allergiques et inflammatoires dans le tractus respiratoire, comprenant administrer la formulation en suspension aérosol par inhalation.

13. Utilisation d'une formulation en suspension aérosol telle que définie dans l'une quelconque des revendications 1 à 9 ou préparée comme il est défini dans l'une quelconque des revendications 10 à 11, dans la préparation d'un médicament pour traiter des réactions allergiques ou inflammatoires dans le tractus respiratoire, comprenant administrer la formulation en suspension aérosol par inhalation.
